# EUROPEAN PATENT APPLICATION

(11) **EP 2 495 231 A1**
(43) Date of publication of application: **05.09.2012**
(21) Application number: 11156389.6
(22) Date of filing: 01.03.2011
(51) Int. Cl.: C07C 29/82, C07C 31/10

(54) **Process for producing highly purified n-propanol by azeotropic distillation**

(71) Applicant: Celanese Chemicals Europe GmbH, 65923 Frankfurt am Main (DE)
(72) Inventor: Blumenstein, Jürgen, 61440 Oberursel (DE); Bauer, Helmut, 65931 Frankfurt am Main (DE)
(74) Representative: von Kreisler Selting Werner

(57) **Abstract**

Disclosed is a process for the production of purified n-propanol having a content of n-propyl propionate of less than 0.003 wt.-% as well as the use of the purified n-propanol thus obtainable for the manufacture of medicaments.
The process comprises the steps:
a) feeding crue n-propanol to a distillation column,
b) separately feeding water to said distillation column,
c) separating an azeotropic mixture which comprises at least water, n-propanol and organic impurities at the top of the distillation column and
d) isolating the purified n-propanol from the bottom of the distillation column.

## Description

This invention relates to a process for the production of purified n-propanol having a content of n-propyl propionate of less than 0.003 wt.-% as well as the use of the purified n-propanol thus obtainable for the manufacture of medicaments.

It is known to produce n-propanol by the hydrogenation of propionaldehyde obtained, for example, by the hydroformylation of ethylene by reaction with carbon monoxide and hydrogen. Usually conventional catalysts such as Raney nickel or Raney cobalt is used for the hydrogenation of propionaldehyde to n-propanol. However, under the hydrogenation conditions undesirably large amounts of certain impurities tend to be formed in the presence of little or no water as a result of side reactions of the hydrogenation reaction. Two of the more significant impurities are n-propyl propionate (prpr) and di-n-propylether (DPE). To reduce the amounts of such impurities formed as a result of these side reactions, the crude propionaldehyde feed to the hydrogenation reaction, if Raney nickel is employed as the hydrogenation catalyst, is adjusted to contain a significant amount of water, e.g., at least about 4.0 % by weight (wt.-%) and up to about 10 wt.-%, based on the weight of the feed to the hydrogenation reaction, resulting in approximately the same percentage of water in the crude n-propanol product from the reaction, based on the weight of such product. However, while this amount of water results in decreased production of prpr and DPE by-products from the hydrogenation reaction, it renders the separation of prpr from n-propanol in the fractioning column more difficult despite the prpr boiling point of about 122 °C, since a prpr/water azeotrope has a boiling point of about 88 °C, giving rise to the presence of liquid prpr above the withdrawal point of purified n-propanol in the column. Moreover, the presence of such a large amount of water in the fractionating column makes the recovery of purified n-propanol from the fractionating column more difficult since n-propanol forms a binary azeotrope with water having a boiling point of about 87 °C, and a ternary azeotrope with water and DPE having a boiling a point of about 74 °C, which must be withdrawn from the column as streams separate from the stream of purified n-propanol having a boiling point of about 97 °C and must be separately purified. Finally, the presence of a relatively large amount of water results in a substantial expenditure of energy, generally through steam consumption, to vaporize the water present, and may also necessitate a larger purification column than would otherwise be necessary to carry out the purification. In view of this, any change in the process is desirable which results in a decreased amount of water necessary in the reactor and the fractionating column and thus a reduction in energy consumption and possibly the size of the column, without any increase in the amount of prpr present in the product which would ordinarily result from the reduced presence of water in the hydrogenation reaction.

US 5866725 discloses a process for the production of purified n-propanol wherein the process comprises contacting in a hydrogenation zone propionaldehyde and hydrogen with an active porous cobalt catalyst, e.g., Raney cobalt, under hydrogenation conditions of temperature and pressure for the production of alcohols from aldehydes, either in the substantial absence of water, or in the presence of water in an amount no more than about 3 wt.-% based on the weight of the resulting crude n-propanol hydrogenation reaction product, and purifying the reaction product by fractional distillation in the substantial absence of water, or in the presence of water in an amount up to about 3 wt.-% of water, based on the total weight of crude n-propanol feed to the fractionating column. However, the process disclosed in US 5,866,725 leads to a purified n-propanol which still comprises about 0.005 wt.-% propyl propionate which might be suitable for various applications, e.g., as a solvent for vegetable oils, waxes, resins, and cellulose esters and ethers in the manufacture of polishing compounds, brake fluids and various propyl compounds. However, the purity requirements for n-propanol which is used in the manufacture of medicaments such as insulin are even higher. For pharmaceutical applications the n-propanol must have a very high degree of purity including a specified low level of certain impurities produced by the hydroformylation and hydrogenation reactions. Especially, for said applications a low level of propyl propionate such as below 0.003 wt.-% is required.

To deal with this problem, CN 101225018 A discloses a method for removing propyl propionate from crude n-propanol which is obtained by hydrogenation of propionaldehyde. According to CN 101225018 A, water is added to the crude n-propanol, and the water containing crude n-propanol is subsequently purified in a fractionation column. However, the content of propyl propionate in the purified n-propanol is still too high, e.g. 0.07 to 0.13 wt.-%.

Thus, there is a demand for a process for the purification of crude n-proponal which meets the requirements for pharmaceutical applications.

It has surprisingly been found that crude n-propanol can effectively be purified and the above-mentioned problems can be solved by the process of the invention.

A first embodiment of the present invention is a process for the production of purified n-propanol having a content of n-propyl propionate of less than 0.003 wt.-% comprising the steps:
a)feeding a crude n-propanol to a distillation column,
b) separately feeding water to said distillation column,
c) separating an azeotropic mixture which comprises at least water, n-propanol and organic impurities at the top of the distillation column and
d) isolating the purified n-propanol from the bottom of the distillation column.

The purified n-propanol which is obtainable by the process of the invention comprises usually less than 0.08 wt.-%, preferably less than 0.05 wt.-% of impurities, based on the total weight of the purified n-propanol mixture. Impurities within the meaning of the present invention are any organic components which are not n-propanol. However, the content of n-propyl propionate in the purified n-propanol is less than 0.003 wt.-%.

According to a preferred embodiment of the invention the overall content of ester in the purified n-propanol is less than 0.005 wt.-%.

The crude n-propanol feed to the process of this invention may be obtained from any source. Generally, n-propanol which is produced in a technical scale is derived from the hydrogenation of propionaldehyde in the presence of catalysts such as Raney nickel or Raney cobalt. The hydrogenation is generally carried out under hydrogenation conditions for the production of alcohols from aldehydes, e.g., a temperature of about 100 °C to about 160 °C, a hydrogen pressure of about 6.8 to about 47.8 bar, and a catalyst loading of about 2 to about 20 wt.-%, preferably about 8 to about 10 wt.-% based on the weight of the liquid components. Therefore, the crude n-propanol which is fed in step a) of the process of the invention is preferably obtainable by the hydrogenation of propionaldehyde. The crude n-propanol which is fed in step a) usually comprises n-propanol in an amount ranging from 90 to less than 99.95 wt.-%, preferably 98 to 99.5 wt.-%, based on the total weight of the crude n-propanol. Depending on the process conditions used during the hydrogenation of the propionaldehyde the crude n-propanol obtained usually comprises more than 0.003 wt.-% n-propyl propionate, preferably between 0.004 wt.-% and 0.5 wt.-%, more preferably between 0.005 wt.-% and 0.1 wt.-%, especially between 0.03 wt.-% and 0.08 wt.-%, based on the weight of the crude n-propanol. According to a preferred embodiment of the invention the crude n-propanol feed is already prepurified by fractional distillation as described in US 5,866,725 or CN 101225018 A, thus leading to a prepurified crude n-propanol having an amount of about 0.005 wt.-% to 0.015 wt.-% n-propyl propionate.

According to a preferred embodiment the amount of water which is separately added to the distillation column is ranging from 1 to 5 wt.-% and preferably ranging from 1.5 to 3.5 wt.-%, based on the total weight of the crude n-propanol feed to the column.

Advantageously, the water which is fed in step b) is purified water, preferably deionized water, more preferably water comprising less than 0.1 wt.-%, especially preferably less than 0.005 wt.-%, e.g. less than 0.002 wt.-% impurities (in conjunction with the purity of water the term "impurity" means every component except water, e.g. mineral salts as well as organic components), based on the weight of the water. It is preferred that the water is obtained from water which has been treated by reverse osmosis or electrical deionization. Especially, if the purified n-propanol which is obtained by the process of the invention is used for the manufacturing of medicaments it is advantageous to used deionized water in order to avoid complexing reactions of mineral salts such as calcium ions with the active components of the medicaments.

The crude n-propanol and the water are fed to a distillation column and distilled. Preferably, the distillation is conducted as a fractional distillation. In a typical fractional distillation, a liquid mixture is heated in the distillation column, preferably at the bottom of the column, and the resulting vapor rises up the fractionating column. The vapor condenses on internal parts which are present in the distillation column, e.g. glass spurs (known as trays or plates) inside the column, and returns to the distilling flask (bottom of the column), refluxing the rising distillate vapor. The hottest tray is at the bottom of the column and the coolest tray is at the top. At steady-state conditions, the vapor and liquid on each tray reach a equilibrium. Only the most volatile of the vapors stays in gas form all the way to the top, where it may then proceed through a condenser, which cools the vapor until it condenses into a liquid distillate. The separation may be enhanced by the addition of more trays (to a practical limitation of heat, flow, etc.).

The term "internal parts present in the distillation column" is understood to mean the components or devices positioned in the column in order to promote and carry out the liquid/vapour exchange. Mention may be made, as examples of internal parts, of structured or unstructured packings, bubble cap plates, valve plates or the like. The internal parts composed of a structured packing are preferred in the process of the invention.

According to a preferred embodiment, a packing material is used in the column instead of trays. This packing material can either be a randomly dumped packing such as Raschig rings or structured metal sheet. Liquids tend to wet the surface of the packing and the vapors pass across this wetted surface, where mass transfer takes place. Differently shaped packings have different surface areas and void space between packings. Both of these factors affect packing performance.

Preferably, the distillation column has a packaging, more preferably a structured packing, especially preferably a structured metal-based packing.

Especially good results in terms of isolation of purified n-propanol could be obtained by fractional distillation wherein the column is packed with stainless steel packing.

In general, the number of trays in the column and the amount of heat transferred to the material being purified in the column are sufficient to produce purified n-propanol containing at least about 99.92 wt.-%, preferably 99.95 wt.-% of n-propanol.

According to a preferred embodiment the distillation column is a fractionating column, preferably having at least 25 trays, more preferably 30 to 60 trays and especially about 50 trays.

The distillation is preferably carried out at atmospheric pressure, although it is possible to operate at subatmospheric or superatmospheric pressures, if desirable under certain circumstances.

It has surprisingly been found that an excellent purification of the crude n-propanol can be achieved in a setup wherein the feed of the crude n-propanol to the distillation column is located above the feed of water.

Especially good results can be obtained wherein the fed crude n-propanol is treated at the boiling point in counter current with the water.

The process of the invention is preferably operated at a continuous steady state.

Unless disturbed by changes in feed, heat, ambient temperature, or condensing, the amount of feed being added usually equals the amount of components being removed.

Typically, a liquid or vapor stream comprising azeotropes of water with n-propanol and/or n-propyl propionate (prpr), all having boiling points in the range of about 74.8 to about 91 °C, are withdrawn from the upper portion of the column at a point above the feed of the crude n-propanol and the feed of water. Purified n-propanol having an atmospheric boiling point of about 97.3 °C is withdrawn at the bottom of the column.

At the top of the column an azeotropic mixture which comprises at least water, n-propanol and impurities can be separated.

The azeotropic mixture separated at the top of the column preferably comprises 65 to 75 wt.-% n-propanol, 25 to 35 wt.-% water and 1 to 4 wt.-% impurities based on the total weight of the azeotropic mixture.

Preferably, the feed with the crude n-propanol is preheated, preferably preheated by heat exchange with purified n-propanol separated from the bottom of the distillation column. Preheating of the crude n-propanol by heat exchange with the purified n-propanol which is separated from the bottom of the column leads to a significant reduction of the energy consumption.

Depending on the impurities present in the crude n-propanol an additional step of purification could be necessary. Preferably, in case the crude n-propanol comprises impurities having a boiling point higher than n-propanol the crude n-propanol can be prepurified by feeding the crude n-propanol to a second distillation column, having preferably 20 to 40 trays, and distilling the n-propanol off which is subsequently feed in step a) of the process of the invention.

Likewise, the purified n-propanol obtained in step d) can be further purified by feeding the n-propanol to a second distillation column and stripping off the n-propanol.

The second rectification column can preferably be used to further increase the purity of the n-propanol. Especially, a second purification step is advisable in order to eliminate traces of odorous components and/or propionaldehyde dipropyl acetal.

Especially, for use of the n-propanol in the manufacturing of medicaments odorous components are not acceptable.

The extremely high grade of purity of the n-propanol obtained by the process of the invention makes it suitable to be used in the manufacturing of medicaments. Especially the extremely low content of n-propyl propionate is advanteous for the use in the manufacturing of medicaments.

A further embodiment of the invention is therefore the use of the purified n-propanol obtainable by the process according to the invention for the manufacture of medicaments.

The purified n-propanol obtainable by the process of the invention is especially suitable for the manufacture of insulin.

Insulin is a hormone that is central to regulating carbohydrate and fat metabolism in the body. Insulin causes cells in the liver, muscle, and fat tissue to take up glucose from the blood, storing it as glycogen in the liver and muscle. Insulin stops the use of fat as an energy source by inhibiting the release of glucagon. Insulin is used as a medicament in the treatment of diabetes.

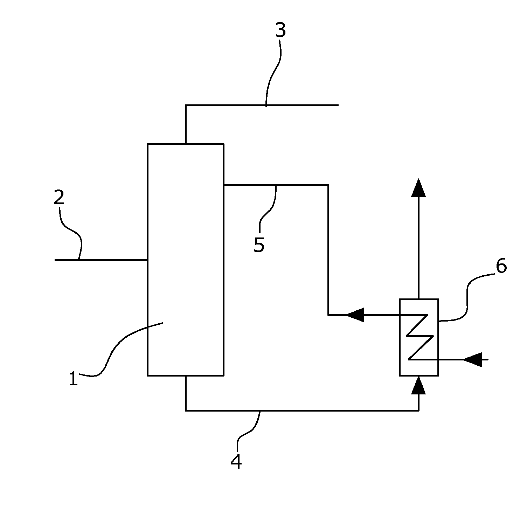

### Brief description of Figure 1

Reference signs:
Distillation column (1)
Feed for water (2)
Outlet for azeotropic mixture (3)
Outlet for purified n-propanol (4)
Feed for crude n-propanol (5)
Heat exchanger (6)

Figure 1 shows a distillation column (1) which can be used for the process of the present invention. The upper part of the column comprises a feed for the crude n-propanol (5). Below the feed for the crude n-propanol (5) at the column the feed for water (2) is arranged. At the top of the column the azeotropic mixture is withdrawn via outlet (3). At the bottom of the column the purified n-propanol is withdrawn via outlet (4). In order to reduce the energy consumption of the purification process the heat of the purified n-propanol withdrawn at the bottom of the column can be used to preheat the crude n-propanol feed (5) by using a heat exchanger (6).

The following non-limiting examples further illustrate the invention.

### EXAMPLE 1

### Example 1 of CN 101225018A (Comparative):

Crude n-propanol with a content of 0.3 wt.-% propyl propionate and 10 wt.-% water is fed to a fractionation column. The temperature at the bottom of the column is adjusted to 90 °C and the temperature at the top of the column is adjusted at 75 °C. The purified n-propanol comprises propyl propionate in an amount of 0.07 wt.-%.

### Example according to the invention:

Crude n-propanol having the following composition:
99.8 wt.-% n-propanol
0.03 wt.-% propyl propionate
0.1 wt.-% water
0.05 wt.-% propionaldehyde dipropyl acetal and
ad 100 wt.-% further organic impurities

The crude n-propanol is fed to a fractionation column having a stainless steel packing and 50 trays with a rate of 1 to/h. Below the feed of the crude n-propanol deionized water is fed to the fractionation column.

The distillation is continuously conducted at a temperature of 97°C to 98°C at the bottom of the column, and a temperature of about 88°C at the top of the column. The azeotropic mixture is continuously stripped off.

The purified n-propanol which could be isolated from the bottom of the column comprises
99.92 wt.-% n-propanol
0.0025 wt.-% propyl propionate
0.005 wt.-% water.

The purified n-propanol obtained is a clear liquid having a typical odor.

## Claims

1. Process for the production of purified n-propanol having a content of n-propyl propionate of less than 0.03 wt.-% comprising the steps:
a) feeding crude n-propanol to a distillation column,
b) separately feeding water to said distillation column,
c) separating an azeotropic mixture which comprises at least water, n-propanol and organic impurities at the top of the distillation column and
d) isolating the purified n-propanol from the bottom of the distillation column.

2. Process according to claim 1 wherein the crude n-propanol which is fed in step a) comprises n-propanol in an amount ranging from 90 to less than 99.95 wt-% n-propanol, based on the total weight of the crude n-propanol.

3. Process according to claim 1 or 2 wherein the crude n-propanol comprises more than 0.003 wt.-% n-propyl propionate, preferably between 0.004 wt.-% and 0.5 wt.-%, more preferably between 0.005 wt.-% and 0.1 wt.-%, especially between 0.03 wt.-% and 0.08 wt.-%, based on the weight of the crude n-propanol.

4. Process according to at least one of the preceding claims wherein the crude n-propanol is obtainable by the hydrogenation of propionaldehyde.

5. Process according to at least one of the preceding claims wherein the water is separately added to the distillation column in an amount ranging from 1 to 5 wt-%, preferably in an amount ranging from 1.5 to 3.5 wt-%, based on the total weight of the crude n-propanol feed.

6. Process according to at least one of the preceding claims wherein the water which is fed in step b) is purified water, preferably deionized water, more preferably water comprising less than 0.1 wt.-%, especially preferably less than 0.005 wt.-%, e.g. less than 0.002 wt.-% impurities.

7. Process according to at least one of the preceding claims wherein the distillation column is a fractionating column, preferably having at least 25 trays, more preferably from 30 to 60 trays, especially about 50 trays.

8. Process according to at least one of the preceding claims wherein the distillation column has a packing, preferably a structured packing, more preferably a structured metal-based packing.

9. Process according to at least one of the preceding claims wherein the distillation is conducted under atmospheric pressure.

10. Process according to at least one of the preceding claims wherein the feed of the crude n-propanol to the distillation column is located above the feed of water.

11. Process according to at least one of the preceding claims wherein the fed crude n-propanol is treated at the boiling point in counter current with the water.

12. Process according to at least one of the preceding claims wherein the process is continuous.

13. Process according to at least one of the preceding claims wherein the azeotropic mixture separated at the top of the column comprises 65 to 75 wt-% n-propanol, 25 to 35 wt-% water and 1 to 4 wt-% impurities, based on the total weight of the azeotrope.

14. Process according to at least one of the preceding claims wherein the feed with the crude n-propanol is preheated, preferably preheated by heat exchange with the purified n-propanol separated from the bottom of the distillation column.

15. Use of the purified n-propanol obtainable by the process according to at least one of the preceding claims for the manufacture of medicaments, preferably insulin.
